# EUROPÄISCHE PATENTSCHRIFT

(11) **EP 3 096 654 B1**
(45) Veröffentlichungstag und Bekanntmachung des Hinweises auf die Patenterteilung: **22.11.2023**
(21) Anmeldenummer: 15702177.5
(22) Anmeldetag: 21.01.2015
(51) Int. Cl.: A61B 5/00, A47C 27/08, A47C 27/10, A47C 31/12, A61B 5/103, A61B 5/11

(54) **VERFAHREN ZUM EINSTELLEN DES DRUCKES IN DEN LUFTKAMMERN EINER MATRATZE**
METHOD FOR ADJUSTING THE PRESSURE IN THE AIR CHAMBERS OF A MATTRESS
PROCÉDÉ POUR AJUSTER LA PRESSION DANS LES CHAMBRES À AIR D'UN MATELAS

(30) Priorität: 23.01.2014 DE 202014100278 U
(43) Veröffentlichungstag der Anmeldung: 30.11.2016
(73) Patentinhaber: Malzl, Hans, 5020 Salzburg (AT)
(72) Erfinder: Malzl, Hans, 5020 Salzburg (AT)
(74) Vertreter: Babeluk, Michael
(86) Internationale Anmeldenummer: PCT/EP2015/051080
(87) Internationale Veröffentlichungsnummer: WO 2015/110448

(56) Entgegenhaltungen:
- WO-A1-2009/102361
- WO-A1-2009/108228
- DE-A1-102004 041 996
- US-A1- 2003 221 261

## Beschreibung

Die Erfindung betrifft ein Verfahren zum Einstellen des Druckes in den Luftkammern einer Matratze aus Schaummaterial, beispielsweise aus Latex oder Schaumstoff, mit eingebetteten, querlaufenden Luftschläuchen, deren Innendruck zum Zweck von verschiedenen liege-physiologischen und therapeutischen Effekten individuell gemessen und verändert werden kann. Ihre Ansteuerung erfolgt über eine in der Matratze integrierte Steuerungseinrichtung, einer sogenannten Technikbox, die über LAN- oder WLAN-Verbindung mit einem Internet-Server oder einem lokalem Rechner verbunden ist und von diesem funktionsrelevante Informationen erhält. Solche Verfahren sind z.B. von WO2009/102361A1 und US2003/221261A1 bekannt.

Eine der wesentlichen Funktionen ist die Fähigkeit der erfindungsgemäßen Matratze, sich durch eine Selbstlern-Intelligenz jenes Stützprofil anzueignen, das zu einem optimierten Schlaf- und Liegeergebnis führt. Diese Selbstlern-Intelligenz wird hier als Ergomatik-Funktion bezeichnet. Dabei wird der Schlaf während der gesamten Nachtruhe in Form von Druckmessungen an bestimmten Körperstellen aufgezeichnet und nach einem Algorithmus in seiner Qualität evaluiert. Hierbei wird der sogenannte Schlaf-Qualitäts-Index, SQI ermittelt.

Ein weiterer Algorithmus löst Veränderungen an der Stützstruktur der Matratze aus, deren Auswirkungen erneut evaluiert werden, sodass das System bei einer Verbesserung der Schlafqualität diesen Weg der systematischen Auslotung fortsetzt bzw. bei einer Verschlechterung einen anderen Versuch unternimmt und der Prozess sich solange wiederholt, bis eine maximal erreichbare Schlafqualität erreicht wurde.

Eine weitere Funktion ist eine so genannte Wechseldruck-Funktion, die dem Phänomen des "Rezeptorischen Wechseleffekts" folgend in vom Benutzer der Matratze zu wählenden Zeitintervallen das Stützprofil der Matratze immer wieder geringfügig verändert, sodass Reizeinwirkungen auf Nervenbahnen vorübergehend entlastet werden, und für den Benutzer der Vorteil einer permanenten Eindämmung von Schmerzen und anderen Akutsymptomen entsteht. Der "Rezeptorische Wechseleffekt" ist dadurch begründet, dass bei geänderter Liegestellung bzw. geändertem Stützprofil der Matratze die der Wirbelsäule entspringenden Spinalnerven nicht in gleichbleibender Weise Druck ausgesetzt sind, sondern dieser sich durch eine leichte Veränderung der Stützstruktur der Matratze ebenfalls verändert bzw. verschiebt. Auf diese Weise werden Spinalnerven, deren Reizung übrigens für die beim Liegen entstehenden Rückenschmerzen verantwortlich sind, in wechselnder Weise mechanisch belastet, sodass jeweils vor dem Erreichen einer Akutsymptomatik diese durch Verschiebung der Druckeinwirkung auf andere Abschnitte der Wirbelsäule abgewendet werden kann. Bei älteren Personen mit entsprechend höherer Reizanfälligkeit ist dieser Wechseleffekt in kürzeren Intervallen vorzunehmen. Dabei kann der Benutzer das Zeitintervall dieser Veränderungen am Stützprofil seiner Matratze selbst wählen, indem er aus verschiedenen angebotenen Optionen wählt.

Ausgehend von dem Standpunkt, dass eine individuell auf ihren Benutzer abgestimmte Matratze zu einem verbesserten Schlaf und zu mehr Erholung führt, haben sich Erfinder und Hersteller in jüngerer Vergangenheit mit dem Bau von individualisierbaren Matratzen beschäftigt. So groß die gesundheitlichen Vorteile derartiger Matratzen auch sein mögen, so schwierig ist es, deren ergonomisch korrektes Stützprofil zu ermitteln und über längere Nutzungszeiträume zu gewährleisten, was schon alleine durch die Abnutzung des Materials, aber auch durch die körperlichen Veränderungen ihres Benutzers erschwert wird.

Die Verwendung von Luft als tragendes und regulierendes Element wurde bereits von vielen Konstrukteuren und Herstellern praktiziert, indem man so genannte Luftbetten auf den Markt brachte, die man heute nicht mehr nur als Einkammer-, sondern auch als Mehrkammerkonzepte findet. Eine individuelle Anpassung war dabei nur in begrenzter und ungenauer Weise möglich und wenn doch, so war die Aufrechterhaltung einer idealen Stützstruktur vom eigenen Eingreifen des Benutzers abhängig, der jedoch in der Regel weder fachlich ausreichend geschult noch erfahren genug ist, um zielführend und erfolgreich agieren zu können.

Im Bereich von Individualmatratzen, deren Regulierung nicht mittels Luftkammern sondern mittels Schaumstoffeinlagen erfolgt, werden zwar bereits Betreuungsdienste angeboten und praktiziert, sodass der Matratzenbenutzer von professionellen Betreuern systematisch zum Erreichen des optimalen Liegeergebnisses geführt wird, allerdings muss der Benutzer selbst die physische Veränderung an seiner Matratze vornehmen, was ein Öffnen der Matratze und den Austausch einzelner Stützelemente erfordert. Eine Maßnahme, die mitunter mühsam sein kann, insbesondere wenn es sich um ältere Personen handelt.

Die gegenständliche Erfindung löst all diese Probleme, indem die Matratze anstelle von eingelegten Stützeinlagen durch unterschiedlich stark befüllte, eng aneinandergereihte Luftkammern ihr Stützprofil erhält, das nachträglich ohne Zutun ihres Benutzers verändert werden kann. Dies einmal in der Weise, dass dem Benutzer - wie schon bei individuellen Schaumstoffmatratzen - ein professioneller Betreuungsdienst zur Verfügung steht, der mit dem Benutzer kommuniziert und die erreichten Liegeergebnisse analysiert und auswertet, dass aber die in der Folge vorzunehmenden Veränderungen am Stützprofil der Matratze nicht vom Benutzer selbst vorgenommen werden müssen, sondern vom Betreuer über eine Internet-Verbindung direkt an die Matratze übermittelt werden, sodass diese ihr Stützprofil entsprechend ändert. Zusätzlich hat der Benutzer erfindungsgemäß die Möglichkeit, anstelle sich von einem Betreuer zum optimalen Liegeergebnis führen zu lassen, diesen Verbesserungsprozess gleich der Matratze selbst zu überlassen, sodass er jene Zeit, die er sonst aufbringen müsste, um mit seinem Betreuer zu kommunizieren, nicht aufbringen muss.

Eine weitere Innovation der erfindungsgemäßen Matratze ist eine Schlaf-Screening-Funktion, die den gesamten Schlafverlauf aufzeichnet, auswertet und dokumentiert. Dazu sind insbesondere in den Luftzuleitungen zu den Luftkammern Drucksensoren eingebaut, die eine Permanentmessung des auf bestimmte Luftkammern einwirkenden Drucks messen, sodass die Steuerungseinrichtung - diese kann sich sowohl innerhalb als auch außerhalb der Matratze befinden - auf Grundlage eines Algorithmus ermittelt, wann Veränderungen der Liegestellung (Seiten-, Rücken- oder Bauchlage) seitens des Benutzers erfolgt sind, wie häufig diese während der gesamten Nachtruhe der Fall waren, und welche Liegestellungen jeweils eingenommen wurden. Dieses Schlaf-Screening bildet die Grundlage einer ebenfalls von der Steuerungseinrichtung mittels eigenem Algorithmus vorgenommenen Berechnung der Schlafqualität bzw. des SQI.

In einer weiteren Ausführung der Erfindung sind im Bereich der Liegefläche, also bei Gebrauchslage im oberen Teil innerhalb der Matratze zusätzlich hochsensible, vorzugsweise flächige Drucksensoren eingearbeitet. Diese bieten die Grundlage für weitere Eigenschaften der erfindungsgemäßen Matratze, wie sie bisher weder bei Matratzen noch bei anderen Konsumprodukten zum Einsatz gebracht wurden.

Erfindungsgemäß sind hierbei Warneinrichtungen vorgesehen, die den Benutzer vor gesundheitlichen Gefahren warnen sollen. Diese gesundheitlichen Gefahren betreffen insbesondere das Phänomen der Schlaf-Apnoe sowie andere Gesundheitsprobleme im Frühstadium, die auf Grundlage von Messungen der Herz- und Atmungsaktivität mit Hilfe der hochsensiblen Drucksensoren im Bereich der Liegefläche der Matratze und deren Vergleich mit zuvor am Rechner abgespeicherten Vergleichsmustern erkennbar sind.

So lassen sich auf Grundlage dieser Herz- und Atmungsmessungen Algorithmen zur Anwendung bringen, die im Sinne eines "häuslichen" Schlaflabors sowohl die Tiefschlaf- als auch die REM-Schlaf-Phasen identifizieren und dabei Anzahl, Zeitpunkt und Länge von Schlaf-Apnoen ermitteln, um bei Überschreiten eines vorgebbaren Grenzwertes einen entsprechenden Alarm auszulösen.

Andere Algorithmen machen es möglich, normale Werte von Herz- und Atmungstätigkeit von anormalen Werten - sie sind in Form von Vergleichsmustern am Rechner bzw. am Server abgespeichert - zu unterscheiden und bei Überschreitung eines Grenzwertes ebenfalls einen Alarm auszulösen. Auf diese Weise wird Krankheitsfrüherkennung erstmals in den häuslichen Bereich der Menschen verlagert, wobei die Signale, die der Körper abgibt, im eigenen Bett und jede Nacht im Sinne einer Permanentbeobachtung, verbunden mit entsprechenden Warnfunktionen, für eine gesundheitliche Frühwarneinrichtung genutzt werden.

Insbesondere ist hierbei zusätzlich vorgesehen, dass die in der Matratze befindlichen, hochsensiblen Drucksensoren mechanischen Druckimpulse in Form von Mikro-Schwingungen, wie sie von Herzschlag und Atmung der auf der Matratze befindlichen Person erzeugt werden, erfassen. So können beispielsweise Frequenz, Intensität, Intensitätsschwankungen, Rhythmik des Herzschlages von diesen hochsensiblen Drucksensoren erfasst werden. Auch die durch die Atmung bedingten mechanischen Druckimpulse im Bereich des Rumpfes können mit diesen Drucksensoren aufgezeichnet werden. Diese Druckimpulse ergeben jeweils Druckprofile, die signifikant für Atmung bzw. Herzschlag sind und die mit zumindest einem zu einem früheren Zeitpunkt abgespeicherten Druckprofil und/oder vorgebbaren Druckprofil verglichen werden. Durch diese Gegenüberstellung von abgespeichertem und aktuellem Druckprofil kann Auskunft erhalten werden, ob eine normale Funktion vorliegt, oder ob eine signifikante Abweichung vorliegt, die auf eine mögliche Krankheitsentwicklung schließen lässt, sodass eine Funktionsauslösung, beispielsweise eine Alarmfunktion erfolgt.

In einer speziell hierfür erstellten Studie des Erfinders wurden die gesammelten Vergleichsmuster, nämlich Druckprofile, in Kategorien unterteilt, um zwischen verschiedenen Krankheitsbildern unterscheiden zu können. Des Weiteren können Verdachtsmomente darüber hinaus in der Weise unterteilt werden, dass sie den Verdacht bereits in eine bestimmte Richtung einer gesundheitlichen Bedrohung lenken, beispielsweise, ob eine Bedrohung in Form von Schlaganfall, Herzinfarkt, Krebs, Diabetes etc. gegeben sein könnte. So kann die Alarmfunktion beispielsweise in Form einer Warnung auch direkt und automatisch an den eigenen Hausarzt mittels Mail weitergeleitet werden, um diesem die Möglichkeit zu geben, anhand einer dem Patienten zugeordneten ärztlichen Auswertungsseite mögliche Verdachtsmomente zu analysieren und auszuwerten, um in der Folge gegebenenfalls dem Matratzenbenutzer - sprich seinem Patienten - eine weiterführende medizinischen Untersuchung anzuraten.

Die von der Matratze gelieferten Druckimpulse hinsichtlich der Atmungsaktivität werden vorzugsweise mit Hilfe eines eigenen Algorithmus in Relation zu den vom Herzschlag gelieferten Mikro-Schwingungen gesetzt, sodass beruhend auf diesen beiden Messgrößen ähnlich einer Navigations-Peilung eine dritte Messgröße ermittelt werden kann, deren Aussagekraft höher ist als die der einzelnen Messgrößen für sich. Auch in diesem Fall werden diese durch Peilverfahren erstellten Messgrößen zu einem Profilmuster umgearbeitet, das mit zumindest einem nach dem gleichen Verfahren in der Studie erstellten und zu einem früheren Zeitpunkt abgespeicherten Vergleichsmuster - sprich Druckprofil - und/oder vorgebbaren Druckprofil verglichen wird, und bei signifikanter Abweichung wiederum eine Funktionsauslösung, beispielsweise eine Warnfunktion erfolgt.

Dieses Peilverfahren wird wahlweise um eine oder mehrere Messgrößen erweitert, beispielsweise um einen Temperaturwert und um biochemische Werte, sodass in diesem Fall auf Grundlage eines eigenen Algorithmus mehr als zwei Messgrößen, nämlich die der Herztätigkeit, die der Atmungstätigkeit, die der Körpertemperatur und jene der gemessenen biochemischen Werte - gewonnen beispielsweise mittels Temperatur-Sensors und/oder Feuchtigkeits-Sensors - eine weitere Messgröße ergeben, deren Aussagekraft nicht nur höher ist als jene der einzelnen Messgrößen für sich alleine, sondern auch höher ist als jene, die im Peilverfahren mit nur zwei Ausgangs-Messgrößen erzielt wird.

Ebenso kann vorgesehen sein, dass die mit Hilfe der Drucksensoren ermittelten Druckprofile über eine medizinische Diagnosesoftware ausgewertet werden, um festzustellen, ob möglicherweise eine krankhafte Veränderung im Schlafverhalten der auf der Matratze befindlichen Person vorliegt.

In einer weiteren Ausführung der Erfindung ist innerhalb der Matratze, vorzugsweise im Bereich der Liegefläche, also im oberen Teil der Matratze zusätzlich zumindest ein hochsensibler Temperatursensor eingearbeitet, der die Körpertemperatur misst. Zudem können bei dieser Ausführung im Inneren der Matratze, vorzugsweise an deren Oberfläche, feine Kapillarschläuche eingearbeitet sein, die mit einer mittels eines außerhalb oder innerhalb der Matratze befindlichen Aggregats bei Bedarf beheizten oder gekühlten Flüssigkeit, vorzugsweise Wasser, befüllt werden. Im Falle erhöhter Körpertemperatur, die auf einen Krankheitszustand schließen lässt, gibt wahlweise der Rechner in der Technikbox der Matratze oder jener des Servers vor, ob und wie lange die Matratze beheizt werden soll. Damit erfährt der Körper des Matratzenbenutzers, ähnlich dem gesundheitlichen Effekt einer Wärmeflasche, von Seiten der Matratze eine wärmeenergetische Unterstützung, die ihm hilft, schneller mit der Krankheit fertig zu werden. Sobald der Körper wieder Normaltemperatur erreicht hat, wird die Matratze ebenfalls wieder auf Normaltemperatur gesetzt. Wahlweise löst das System bei Überschreiten der Körpernormaltemperatur eine Alarmfunktion aus.

Das Verfahren zur Ermittlung der Körpertemperatur bzw. dem Überschreiten des normalen Wertes beruht darauf, dass das System der Matratze permanente Temperaturmessungen vornimmt, aus deren Durchschnitt die Körpernormaltemperatur mittels Algorithmus ermittelt wird. Dadurch kann der Unsicherheitsfaktor, der sich aus dem Umstand ergibt, dass jeder Körper in Relation zu dem ihn umgebenden Umfeld - sprich Zudecke, Matratzenmaterial, Kleidung und Raumtemperatur - eine unterschiedliche Körpertemperatur aufweist, ausgeschlossen werden. Ein über einen längeren Zeitpunkt errechneter Durchschnitts-Temperaturwert bildet damit die Berechnungsgrundlage für die Ermittlung der normalen Körpertemperatur, sodass danach auf Basis dieses normalen Wertes ein Überschreiten der normalen Körpertemperatur sowie auch der graduelle Wert dieser Überschreitung ermittelt werden kann.

In einer weiteren Ausführung der Erfindung sind innerhalb der Matratze, vorzugsweise im Bereich der Liegefläche, also im oberen Teil der Matratze zusätzliche hochsensible Feuchtigkeitssensoren eingearbeitet, mit deren Hilfe das System in der Lage ist, über die vom Körper abgegebene Feuchtigkeit - sprich Schweiß - Auskunft hinsichtlich biochemischer Parameter zu geben, die ihrerseits Indikatoren für einen bestimmten Gesundheitszustand sind. Diese können in die Erarbeitung der Vergleichsmuster aufgenommen werden und zusätzlich weitere Messwerte für das oben beschriebene Peilverfahren liefern.

Im Folgenden wird anhand von nicht-einschränkenden Ausführungsbeispielen mit zugehörigen Figuren die Erfindung näher erläutert.

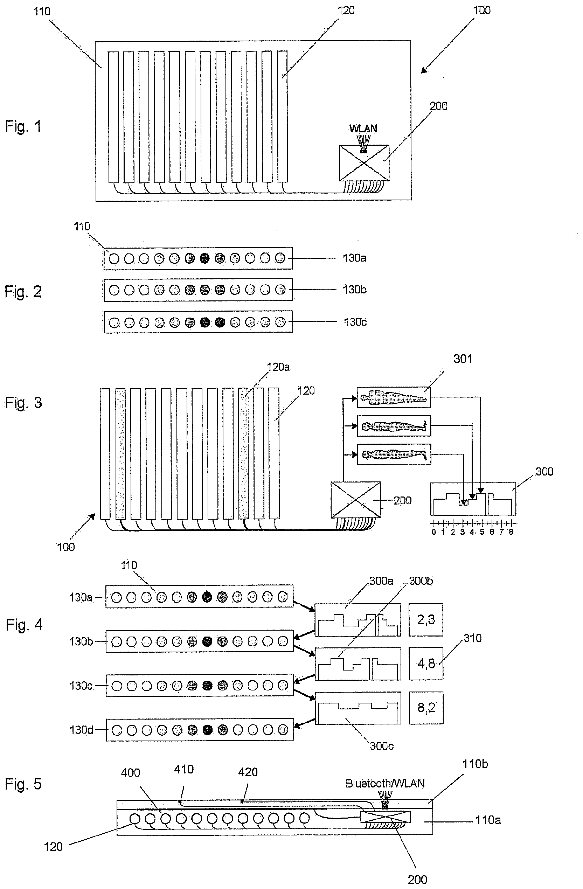

Figur 1 zeigt eine erfindungsgemäße Vorrichtung 100 mit aneinander gereihten, in Querrichtung in die Matratze 110 eingearbeitete Luftkammern 120, die von einer Steuerungseinrichtung 200 zum Zwecke der Befüllung mit Luft angesteuert sind. Die hierfür benötigten Drucksensoren sind hierbei in der Steuereinheit 200 angeordnet. Eine LAN- oder WLAN-Verbindung übermittelt die Befehle dazu.
Figur 2 zeigt drei unterschiedliche Druck- bzw. Stützprofile 130a, 130b, 130c der Matratze 110, wie sie beispielsweise bei deren Wechseldruck-Funktion in entsprechend gesetzter Abfolge von der Matratze 110 selbständig gebildet werden.
Figur 3 zeigt in einer schematischen Darstellung das erfindungsgemäße Verfahren mit der Schlaf-Screening-Funktion. Zu sehen sind die einzelnen Luftkammern 120, wovon in der dargestellten Variante der Erfindung zwei Luftkammern 120a eine Permanentmessung des Luftdrucks innerhalb der Luftkammer 120a ausüben. Die dabei gemessenen Druckwerte werden an die Steuerungseinrichtung 200 übermittelt, die anhand eines vorab vom Matratzenbenutzer erstellten Liegeprofils 300 in den unterschiedlichen Liegestellungen 301 einen Abgleich vornimmt und auf diese Weise die zum jeweiligen Zeitpunkt eingenommene Liegestellung 301 ermitteln kann.
Figur 4 zeigt in einer schematischen Darstellung das erfindungsgemäße Verfahren mit Ergomatik-Funktion, wobei die Matratze 110 nach einem Algorithmus gesteuert ihr Druck- bzw. Stützprofil 130a, 130b, 130c, 130d in geringem Umfang verändert, danach eine Evaluierung der Schlafqualität über die erfassten Liegeprofile 300a, 300b, 300c vorgenommen und in der Folge bewertet wird, ob die Veränderung des Stützprofils 130a, 130b, 130c, 130d zu einer Verbesserung der Schlafqualität geführt hat oder nicht. Diese Verbesserung der Schlafqualität wird in Form eines numerischen Wertes 310, dem sogenannten Schlaf-Qualität-Index (SQI) ausgegeben, der über den Algorithmus in Zusammenhang mit der Positionsänderung des Benutzers steht. Diese Erkenntnis löst eine weitere Veränderung des Stützprofils 130a, 130b, 130c, 130d aus und der Vorgang wiederholt sich solange, bis ein Maximum an Schlafqualität erreicht ist.

In der Figur 5 ist eine weitere Ausführung der Erfindung dargestellt, wobei die erfindungsgemäße Matratze 110 in einem Matratzenunterteil 110a Luftkammern 120 aufweist, deren Innendruck auf die bereits oben beschriebene Weise verändert werden kann, um das für eine optimale Schlafqualität erforderliche Stützprofil 300 zu erhalten. In einem die Liegefläche bildenden Matratzenoberteil 110b ist eine flächige Druckmesseinrichtung 400 angeordnet, die aus einer Vielzahl von hochsensiblen Druckmesssensoren besteht, die in der Lage sind, die von Atmung und Herzschlag einer auf der Matratze 110 liegenden Person erzeugten Mikroschwingungen zu erfassen und über die Steuerungseinrichtung 200 direkt oder über ein Smartphone, Tablet oder an eine andere Rechnereinrichtung an einen Internetserver übermittelt werden, wobei die Übertragung der Druckimpulse beispielsweise über WLAN oder Bluetooth erfolgen kann.

Des Weiteren sind in dieser Ausführung der Erfindung in dem Matratzenoberteil 110b weitere Sensoren, nämlich ein Temperatursensor 410 und ein Feuchtigkeitssensor 420 angeordnet, die der Erfassung von Parametern wie der Köpertemperatur oder biochemischen Parametern dienen, wie sie von den Algorithmen zur Früherkennung von Krankheiten genutzt werden.

## Patentansprüche

1. Verfahren zum Einstellen des Druckes in den Luftkammern (120, 120a) einer Matratze (110) aus elastischem Schaum- bzw. Gummimaterial mit in Querrichtung eingelegten und einzeln ansteuerbaren Luftkammern (120, 120a), wobei der Druck in den Luftkammern (120, 120a) auf Grundlage eines von einer Steuerungseinrichtung angewandten Algorithmus eingestellt wird, wobei über den Zeitraum einer Nachtruhe mittels Druckmess-Sensoren Druckprofile (300a, 300b, 300c) aufgezeichnet werden, die anschließend ausgewertet werden, ob sich die liegende Person in einer bestimmten Liegestellung wie beispielsweise in Seiten-, Rücken- oder Bauchlage befindet, wobei bevorzugterweise die Zuordnung der ermittelten Liegestellungen zeitlich erfolgt, sowohl was die Lageveränderung als auch die Dauer der jeweils eingenommenen Liegestellung betrifft, wobei Druckimpulse von in einer Matratze (11) vorzugsweise flächig und im Verbund angeordneten Drucksensoren aufgenommen werden, und ein auf Basis der Druckimpulse erstelltes aktuelles Druckprofil (300a, 300b, 300c) erstellt wird, und dieses aktuelle Druckprofil (300a, 300b, 300c) mit zumindest einem zu einem früheren Zeitpunkt erstellten Druckprofil (300a, 300b, 300c) verglichen wird, und bei Abweichung von einem vorgebbaren Wert oder Wertebereich eine Funktionsauslösung erfolgt, und wobei der angewandte Algorithmus in Wechselwirkung zu von der Steuerungseinrichtung vorgenommenen Schlafqualitätsberechnungen steht und/oder das Prinzip des Rezeptorischen Wechseldrucks verfolgt, **dadurch gekennzeichnet, dass** auf Grundlage eines Algorithmus ein gemessener Druckwert in Relation zu mindestens einem anderen Druckwert des in der Matratze eingebauten Drucksensors gebracht wird und der dadurch entstehende Wert eine neue, dritte und dadurch aussagekräftigere Messgröße ergibt, wobei die gewonnene dritte Messgröße in Relation zum gemessenen Wert eines Temperatursensors und/oder in Relation zum gemessenen Wert eines Feuchtigkeitssensors gebracht wird.

2. Verfahren nach Anspruch 1, **dadurch gekennzeichnet, dass** die Funktionsauslösung eine Warnfunktion ist oder die Funktionsauslösung eine Veränderung des Druckes in Luftkammern (120, 120a) der Matratze (110) ist, um eine Veränderung der Stützfunktion der Matratze (110) für eine darauf befindlichen Person zu erzielen.

3. Verfahren nach Anspruch 1 oder 2, **dadurch gekennzeichnet, dass** Druckprofile (300a, 300b, 300c) des jeweiligen Matratzenbenutzers abgespeichert werden.

4. Verfahren nach einem der Ansprüche 1 bis 3, **dadurch gekennzeichnet, dass** die Steuerungseinrichtung über eine Internet-Verbindung von zumindest einem Server Daten für den anzuwendenden Algorithmus erhält.

5. Verfahren nach einem der Ansprüche 1 bis 4, **dadurch gekennzeichnet, dass** über zumindest einem Temperatursensor die aktuelle Körpertemperatur einer auf der Matratze (110) befindlichen Person mithilfe eines Algorithmus errechnet wird, die anschließend mit einer auf Grundlage einer Langzeitmessung zu einem früheren Zeitpunkt ermittelten Körpernormaltemperatur verglichen wird.

6. Verfahren nach einem der Ansprüche 1 bis 5, **dadurch gekennzeichnet, dass** bei Abweichung der aktuellen Körpertemperatur von der Körpernormaltemperatur, insbesondere bei Vorliegen einer Temperaturerhöhung, eine Temperierung der Matratze (110) über eine Temperierungseinrichtung erfolgt oder eine Warnfunktion ausgelöst wird.

## Claims

1. Method for adjusting the pressure in the air chambers (120, 120a) of a mattress (110) made of elastic foam or rubber material with air chambers (120, 120a) which are inserted in the transverse direction and are individually controllable, wherein the pressure in the air chambers (120, 120a) is set on the basis of an algorithm used by a control device, wherein pressure profiles (300a, 300b, 300c) are recorded over the period of a night's rest by means of pressure measurement sensors, which pressure profiles are then evaluated to determine whether the person lying on the mattress is in a specific lying position such as, for example, in the lateral, supine or prone position, wherein preferably the assignment of the determined lying positions is carried out temporally, both with regard to the change in position and the duration of the lying position assumed in each case, wherein pressure pulses are recorded by pressure sensors arranged in a mattress (11), preferably in a planar and composite manner, and a current pressure profile (300a, 300b, 300c) is created on the basis of the pressure pulses, and this current pressure profile (300a, 300b, 300c) is compared with at least one pressure profile (300a, 300b, 300c) created at an earlier point in time, and in the event of a deviation from a predeterminable value or range of values, a function triggering is carried out, and wherein the algorithm used interacts with sleep quality calculations performed by the control device and/or follows the principle of receptor alternating pressure, **characterised in that,** on the basis of an algorithm, a measured pressure value is brought into relation with at least one other pressure value of the pressure sensor installed in the mattress and the resulting value yields a new, third and thus more meaningful measured variable, wherein the obtained third measured variable is brought into relation with the measured value of a temperature sensor and/or into relation with the measured value of a humidity sensor.

2. Method according to claim 1, **characterised in that** the function triggering is a warning function or the function triggering is a change in pressure in air chambers (120, 120a) of the mattress (110) to achieve a change in the support function of the mattress (110) for a person placed thereon.

3. Method according to claim 1 or 2, **characterised in that** pressure profiles (300a, 300b, 300c) of the respective mattress user are stored.

4. Method according to one of claims 1 to 3, **characterised in that** the control device receives data for the algorithm to be applied from at least one server via an internet connection.

5. Method according to one of claims 1 to 4, **characterised in that** the current body temperature of a person located on the mattress (110) is calculated via at least one temperature sensor with the aid of an algorithm, which is subsequently compared with a normal body temperature determined on the basis of a long-term measurement at an earlier time.

6. Method according to one of claims 1 to 5, **characterised in that** if the current body temperature deviates from the normal body temperature, in particular if there is an increase in temperature, the temperature of the mattress (110) is controlled by a temperature-control device or a warning function is triggered.

## Revendications

1. Procédé de réglage de la pression dans les chambres à air (120, 120a) d'un matelas (110) en mousse ou caoutchouc élastique avec des chambres à air (120, 120a), orientées transversalement et se commandant individuellement, selon lequel
la pression se règle dans les chambres à air (120, 120a) avec un algorithme appliqué par une installation de commande,
sur une durée d'un repos nocturne, on enregistre les profils de pression (300a, 300b, 300c) pris par des capteurs de mesure de pression, et qui sont exploités selon que la personne couchée se trouve dans une certaine position telle que, par exemple, sur le côté, sur le dos ou sur le ventre, de façon préférentielle, l'association de la position couchée, déterminée se faisant dans le temps, à la fois pour le changement de position que pour la durée de la position couchée respective,
on prend des impulsions de pression de capteurs de pression installés, de préférence, en surface et en combinaison dans un matelas (11) on établit un profil de pression (300a, 300b, 300c) actuel, établi en se fondant sur les impulsions de pression et on compare ce profil de pression actuel (300a, 300b, 300c) à au moins un profil de pression (300a, 300b, 300c) établi antérieurement et en cas d'écart par rapport à une valeur prédéfinie ou une plage de valeurs, on déclenche une fonction, l'algorithme appliqué étant en interaction avec les calculs de qualité de sommeil effectués par l'installation de commande et/ou le principe de la pression alternée prescrite,
**caractérisé en ce que**
sur le fondement de l'algorithme on met une valeur de pression mesurée en relation avec au moins une autre valeur de pression du capteur de pression installé dans le matelas et à partir de la valeur obtenue, on a une nouvelle troisième grandeur de mesure ainsi plus significative,
la troisième grandeur de mesure obtenue étant mise en relation avec la valeur mesurée d'un capteur de température et/ou en relation avec la valeur mesurée d'un capteur d'humidité.

2. Procédé selon la revendication 1,
**caractérisé en ce que**
le déclenchement de la fonction est une fonction d'avertissement ou une fonction de déclenchement d'une modification de la pression dans les chambres à air (120, 120a) du matelas (110) pour obtenir une variation de la fonction de support du matelas (110) pour la personne installée sur celui-ci.

3. Procédé selon la revendication 1 ou 2,
**caractérisé en ce que**
on enregistre les profils de pression (300a, 300b, 300c) de l'utilisateur respectif du matelas.

4. Procédé selon l'une des revendications 1 à 3,
**caractérisé en ce que**
l'installation de commande reçoit par une liaison par internet, d'au moins un serveur, des données pour l'algorithme à appliquer.

5. Procédé selon l'une des revendications 1 à 4,
**caractérisé en ce que**
on calcule avec au moins un capteur de température, la température corporelle actuelle d'une personne sur le matelas (110) avec un algorithme et ensuite on le compare à une température normale du corps, obtenue à partir d'une mesure à long terme faite antérieurement.

6. Procédé selon l'une des revendications 1 à 5,
**caractérisé en ce que**
en cas d'écart de la température corporelle actuelle par rapport à la température corporelle normale, notamment en cas d'élévation de la température, on déclenche une mise en température du matelas (110) par une installation de mise en température ou on déclenche une fonction d'avertissement.
